(19) Europäisches Patentamt
European Patent Office
Office européen des brevets

(11) Veröffentlichungsnummer : **0 068 352**
**B1**

(12) # EUROPÄISCHE PATENTSCHRIFT

(45) Veröffentlichungstag der Patentschrift : **09.01.85**

(51) Int. Cl.⁴ : **C 11 D 1/29**, C 11 D 1/12

(21) Anmeldenummer : **82105352.7**

(22) Anmeldetag : **18.06.82**

(54) **Tensidmischungen aus alpha-Olefinsulfonaten und anderen Tensiden.**

(30) Priorität : **26.06.81 DE 3125102**

(43) Veröffentlichungstag der Anmeldung : **05.01.83 Patentblatt 83/01**

(45) Bekanntmachung des Hinweises auf die Patenterteilung : **09.01.85 Patentblatt 85/02**

(84) Benannte Vertragsstaaten : **AT BE CH DE FR GB IT LI NL SE**

(56) Entgegenhaltungen :
**DE-A- 2 146 373**
**DE-A- 2 638 901**

(73) Patentinhaber : **HOECHST AKTIENGESELLSCHAFT**
**Postfach 80 03 20**
**D-6230 Frankfurt am Main 80 (DE)**

(72) Erfinder : **Täuber, Günther, Dr.**
**Rauenthaler Weg 32**
**D-6000 Frankfurt am Main 71 (DE)**
Erfinder : **Skrypzak, Werner**
**Eichkopfallee 33**
**D-6237 Liederbach (DE)**
Erfinder : **Reng, Alwin**
**Im Schulzehnten 22**
**D-6233 Kelkheim (Taunus) (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Zur Reinigung von verschmutzten Oberflächen werden bekannterweise anionische, nichtionische, kationische und amphotere Tenside, allein oder in Kombination mit den verschiedensten Hilfsstoffen, die den Reinigungseffekt unterstützen, angewandt.

Bekannt ist der Einsatz von α-Olefinsulfonaten zur Herstellung von schäumenden Reinigungsmitteln für technische und kosmetische Zwecke. Im allgemeinen handelt es sich dabei um langkettige Alkenylsulfonate oder langkettige Hydroxyalkansulfonate, deren Herstellung in zahlreichen Patenten und Publikationen beschrieben ist. Aus kommerziellen Gründen werden meist die entsprechenden Natriumsalze eingesetzt. Für solche Reinigungsmittel wird in der Praxis aus Gründen der besseren Verteilbarkeit, der günstigeren Dosierung und Handhabung meist eine hohe Viskosität gewünscht. Es zeigte sich jedoch, daß bei der Formulierung von Reinigungspräparaten mit α-Olefinsulfonaten eine Erhöhung der Viskosität mit den praxisüblichen und preiswerten Elektrolyten, wie Natriumchlorid, Ammoniumchlorid, Kaliumchlorid, Magnesiumsulfat und Harnstoff sehr schwierig ist.

Es wurde nun gefunden, daß man durch Zugabe von Salzen dann die gewünschten hohen Viskositätswerte erreichen kann, wenn man anstelle von reinen α-Olefinsulfonaten von einer Mischung dieser α-Olefinsulfonate mit bestimmten anderen Tesiden ausgeht. Außerdem läßt sich mit diesen Mischungen eine Verbesserung des Schaumverhaltens erreichen.

Gegenstand der Erfindung sind somit Tensidmischungen bestehend aus 20 bis 80, vorzugsweise 30 bis 70 Gew.-% eines $C_{12}$-$C_{20}$ α-Olefinsulfonats und 20 bis 80, vorzugsweise 30 bis 70 Gew.-% einer oder mehrerer Verbindungen der Formeln

$$RCONH-(CH_2CH_2O)_n-SO_3X \quad (1)$$

wobei R $C_7$-$C_{17}$-Alkyl, n eine Zahl von 0 bis 4 und X ein Alkalimetall-, Erdalkalimetall- oder Alkanolammoniumion bedeutet,

$$RCON(CH_3)CH_2CH_2SO_3X \quad (2)$$

wobei R und X die gleiche Bedeutung haben wie in Formel (1),

$$R_1O(CH_2CH_2O)_nCOCH_2-CH(SO_3Y)-COOY \quad (3)$$

worin $R_1$ $C_{12}$-$C_{14}$ Alkyl, n eine Zahl von 0 bis 5 und Y ein Alkalimetallion bedeutet,

$$R_2CONH-(CH_2)_n-{}^{\oplus}N(R_3)(R_3)-CH_2COO^{\ominus} \quad (4)$$

worin $R_2$ $C_{10}$-$C_{14}$-Alkyl, n 2 oder 3 und $R_3$ $C_1$-$C_4$-Alkyl bedeutet,

$$R_4-N(R_3)(R_3)=O \quad (5)$$

worin $R_4$ $C_{10}$-$C_{18}$-Alkyl und $R_3$ $C_1$-$C_4$-Alkyl bedeutet und

$$R_4CONH(CH_2)_n-N(R_3)(R_3)=O \quad (6)$$

worin $R_4$ $C_{10}$-$C_{18}$-Alkyl, n 2 oder 3 und $R_3$ $C_1$-$C_4$-Alkyl bedeutet.

Bei den erfindungsgemäß zu verwendenden α-Olefinsulfonaten handelt es sich um Produkte, die durch Umsetzung von $C_{12}$-$C_{18}$ Olefinen mit Schwefeltrioxid erhalten werden und die im wesentlichen aus Alkensulfonaten und Hydroxyalkansulfonaten bestehen. Bei den Verbindungen der obigen Formel 1 handelt es sich um Acylaminopolyglykolethersulfate, wie sie in « Seifen-Öle-Fette-Wachse » S. 307-311, (1976) beschrieben sind. Die Herstellung der Verbindungen der Formeln 2 erfolgt durch Umsetzung von Oxyäthansulfosäure, N-Methyltaurin mit Fettsäurechloriden (Parfümerie und Kosmetik 42, S. 203-206 (1961). Die Herstellung der Betaine der Formel 4 ist in der DE-B 29 26 479 beschrieben.

Die Herstellung der Mischungen aus α-Olefinsulfonat und einem oder mehreren der anderen Tenside erfolgt durch einfaches Vermischen, gegebenenfalls unter leichtem Erwärmen. Um die Viskosität dieser Mischungen noch zu erhöhen, kann man noch Neutralsalze zugeben wie Ammoniumchlorid, Kaliumchlorid, Magnesiumsulfat oder bevorzugt Natriumchlorid oder auch Harnstoff in Mengen von 5-15, vorzugsweise 10 Gew.-%. Diese Tensidmischungen werden schäumenden Reinigungsmitteln für technische und insbesondere für kosmetische Zwecke zugegeben wie beispielsweise Haarshampoos, Schaumbäder, Duschbäder, Geschirrspülmittel, flüssige Waschmittel, Allzweckreiniger, Autoshampoos, und andere flüssige Spül-, Wasch-, und Reinigungsmittel. Der Gehalt an den erfindungsgemäßen Tensidmischungen in diesen Reinigungsmitteln beträgt ca. 5 bis 15, vorzugsweise 10 Gew.-%.

Die Prüfung dieser Mischungen erfolgte nach folgenden Kriterien :

1. Schaumverhalten

In Anlehnung an die Methode nach ROSS-MILES wurde gemessen wie in « Parfümerie und Kosmetik 45 » Seite 59 (1964) beschrieben, jeweils in einer Konzentration von 0,03 % Tensid, bzw. Tensidmischung, gelöst in Wasser. Abgelesen wurde die Schaumhöhe sofort nach Ende des Schaumerzeugungsvorganges. Die Messungen wurden bei + 20 °C und mit Wasser mit einer Härte von 20° dH durchgeführt.

2. Äußeres

Visuell wurde das Äußere der Tensidmischungen, die auf einen Gehalt von 15 % waschaktiver Substanz eingestellt wurden, nach 4-wöchiger Lagerung bei + 20 °C beurteilt. Das äußere Erscheinungsbild wurde in klar, etwas trüb und trüb klassifiziert.

3. Viskosität

Die Bestimmung der Viskosität erfolgte mit dem BROOKFIELD-Viskosimeter, Type RVT bzw. LVT bei + 20 °C und nach folgender Klassifizierung :

1- 200 mPas = dünnflüssig
200- 1 000 mPas = niedrigviskos
1 000- 5 000 mPas = mittelviskos
5 000-20 000 mPas = hochviskos
über 20 000 mPas = Gel

Die Komponente A in allen Beispielen ist $C_{12}$-$C_{20}$-α-Olefinsulfonat. In der Spalte « Viskosität » ist neben dem jeweiligen Viskositätsbereich in Klammern die dafür jeweils erforderliche Menge an Kochsalz angegeben.

Beispiel 1

Komponente B = Verbindung der Formel 1 mit R = $C_{11}$-$C_{13}$-Alkyl, n = 3 und X = Na

| Mischungsverhältnis Komponente A : B | Schaumhöhe | Viskosität | | Äußeres |
|---|---|---|---|---|
| 100 : 0 | 190 | mittelviskos | (10 %) | etwas trüb |
| 70 : 30 | 200 | mittelviskos | (14 %) | trüb |
| 50 : 50 | 195 | mittelviskos | (12 %) | klar |
| 30 : 70 | 185 | niedrig viskos | (12 %) | klar |
| 0 : 100 | 160 | dünnflüssig | (13 %) | klar |

Die Mischungen haben eine wesentlich bessere Schaumwirkung und eine höhere Viskosität im Vergleich zur reinen Komponente B. Gegenüber der reinen Komponente A sind die Mischungen klarer.

Beispiel 2

Komponente B = Verbindung der Formel 2 mit R = Palmkernfett-Alkyl und X = Na

| Mischungsverhältnis Komponente A : B | Schaumhöhe | Viskosität | | Äußeres |
|---|---|---|---|---|
| 100 : 0 | 190 | mittelviskos | (10 %) | etwas trüb |
| 70 : 30 | 195 | hochviskos | (12 %) | etwas trüb |
| 50 : 50 | 190 | hochviskos | (10 %) | etwäs trüb |
| 30 : 70 | 185 | mittelviskos | (10 %) | trüb |
| 0 : 100 | 185 | dünnflüssig | (10 %) | trüb |

Im Vergleich zur reinen Komponente B sind die Mischungen sehr viel besser in ihrer Viskosität und auch etwas klarer. Eine Viskositätserhöhung ist auch gegenüber der reinen Komponente A zu beobachten.

Beispiel 3

Komponente B = Verbindung der Formel 3 mit $R_1$ = Lauryl, n = 3 und Y = Na

| Mischungsverhältnis Komponente A : B | Schaumhöhe | Viskosität | | Äußeres |
|---|---|---|---|---|
| 100 : 0 | 190 | mittelviskos | (10 %) | etwas trüb |
| 70 : 30 | 180 | hochviskos | (11 %) | klar |
| 50 : 50 | 165 | hochviskos | (13 %) | klar |
| 0 : 100 | 115 | dünnflüssig | (10 %) | klar |

Die Mischungen zeigen hier deutlich höhere Viskositätswerte im Vergleich zu den reinen Komponenten A und B.

Beispiel 4

Komponente B = Verbindung der Formel 4 mit $R_2$ = Cocosfett-alkyl, n = 2 und $R_3 = CH_3$

| Mischungsverhältnis Komponente A : B | Schaumhöhe | Viskosität | | Äußeres |
|---|---|---|---|---|
| 100 : 0 | 190 | mittelviskos | (10 %) | etwas trüb |
| 70 : 30 | 175 | hochviskos | ( 6 %) | klar |
| 50 : 50 | 170 | Gel | ( 3 %) | klar |
| 30 : 70 | 170 | Gel | ( 2 %) | klar |
| 0 : 100 | 165 | dünnflüssig | (13 %) | klar |

Man erhält hier klare Lösungen, die durch Zugabe von nur geringen Mengen Kochsalz sehr hohe Viskositätswerte bis in den Gel-Bereich zeigen.

Beispiel 5

Komponente B = Verbindung der Formel 5 mit $R_4 = C_{12}$-$C_{14}$-Alkyl, $R_3 = CH_3$

| Mischungsverhältnis Komponente A : B | Schaumhöhe | Viskosität | | Äußeres |
|---|---|---|---|---|
| 100 : 0 | 190 | mittelviskos | (10 %) | etwas trüb |
| 70 : 30 | 170 | hochviskos | ( 6 %) | klar |
| 50 : 50 | 195 | hochviskos | ( 3 %) | klar |
| 30 : 70 | 195 | hochviskos | ( 4 %) | klar |
| 0 : 100 | 185 | dünnflüssig | (10 %) | klar |

Diese Beispiel zeigt deutlich, daß die Mischungen, im Vergleich zu den neuen Komponenten A und B, eine wesentlich höhere Viskosität aufweisen bei gleichzeitiger Erniedrigung des Zusatzes am Elektrolyt.

**Ansprüche** (für die Vertragsstaaten : BE, CH, DE, FR, GB, IT, LI, NL, SE)

1. Tensidmischungen dadurch gekennzeichnet, daß sie bestehen aus 20 bis 80 Gew.-% eines $C_{12}$-$C_{20}$-α-Olefinsulfonats und 20 bis 80 Gew.-% einer oder mehrerer Verbindungen der Formeln

$$RCONH-(CH_2CH_2O)_n-SO_3X \qquad (1)$$

wobei R $C_7$-$C_{17}$-Alkyl, n eine Zahl von 0 bis 4 und X ein Alkalimetall-, Erdalkalimetall- oder Alkanolammoniumion bedeutet,

$$RCO\overset{CH_3}{N}CH_2CH_2SO_3X \qquad (2)$$

wobei R und X die gleiche Bedeutung haben wie in Formel (1),

$$R_1O(CH_2CH_2O)_nCOCH_2-\underset{SO_3Y}{CH}-COOY \qquad (3)$$

worin $R_1$ $C_{12}$-$C_{14}$-Alkyl, n eine Zahl von 0 bis 5 und Y ein Alkalimetallion bedeutet,

$$R_2CONH-(CH_2)_n-\overset{R_3}{\underset{R_3}{\overset{\oplus}{N}}}-CH_2COO^{\ominus} \qquad (4)$$

worin $R_2$ $C_{10}$-$C_{14}$-Alkyl, n 2 oder 3 und $R_3$ $C_1$-$C_4$-Alkyl bedeutet,

$$R_4 - \overset{R_3}{\underset{R_3}{N}} = O \qquad (5)$$

worin $R_4$ $C_{10}$-$C_{18}$-Alkyl und $R_3$ $C_1$-$C_4$-Alkyl bedeutet und

$$R_4CONH(CH_2)_n - \overset{R_3}{\underset{R_3}{N}} = O \qquad (6)$$

worin $R_4$ $C_{10}$-$C_{18}$-Alkyl, n 2 oder 3 und $R_3$ $C_1$-$C_4$-Alkyl bedeutet.

2. Tensidmischungen nach Anspruch 1, dadurch gekennzeichnet, daß sie bestehen aus 30 bis 70 Gew.-% des α-Olefinsulfonats und 30 bis 70 Gew.-% einer oder mehrerer Verbindungen der Formeln (1) bis (6).

**Ansprüche** (für den Vertragsstaat AT)

1. Verfahren zur Herstellung von Tensidmischungen durch Vermischen eines α-Olefinsulfonats mit wenigstens einem zweiten Tensid, dadurch gekennzeichnet, daß man 20 bis 80 Gew.-% eines $C_{12}$-$C_{20}$-α-Olefinsulfonats mit 20 bis 80 Gew.-% einer oder mehrerer Verbindungen der Formeln

$$RCONH-(CH_2CH_2O)_n-SO_3X \qquad (1)$$

wobei R $C_7$-$C_{17}$-Alkyl, n eine Zahl 0 bis 4 und X ein Alkalimetall-, Erdalkalimetall- oder Alkanolammoniumion bedeutet,

$$RCON(CH_3)CH_2CH_2SO_3X \quad (2)$$

worin R und X die gleiche Bedeutung haben wie in Formel (1),

$$R_1O(CH_2CH_2O)_nCOCH_2-CH(SO_3Y)-COOY \quad (3)$$

worin $R_1$ $C_{12}$-$C_{14}$-Alkyl, n eine Zahl 0 bis 5 und Y ein Alkalimetallion bedeutet,

$$R_2CONH-(CH_2)_n-{}^{\oplus}N(R_3)_2-CH_2COO^{\ominus} \quad (4)$$

worin $R_2$ $C_{10}$-$C_{14}$-Alkyl, n 2 oder 3 und $R_3$ $C_1$-$C_4$-Alkyl bedeutet,

$$R_4-N(R_3)_2=O \quad (5)$$

worin $R_4$ $C_{10}$-$C_{18}$-Alkyl und $R_3$ $C_1$-$C_4$-Alkyl bedeutet und

$$R_4CONH(CH_2)_n-N(R_3)_2=O \quad (6)$$

worin $R_4$ $C_{10}$-$C_{18}$-Alkyl, n 2 oder 3 und $R_3$ $C_1$-$C_4$-Alkyl bedeutet, vermischt.

2. Verfahren nach Anspruch 1, dadurch gekennzeichnet, daß man 30 bis 70 Gew.-% des α-Olefinsulfonats mit 30 bis 70 Gew.-% einer oder mehrerer Verbindungen der Formeln 1 bis 6 vermischt.

**Claims** (for the Contracting States : BE, CH, DE, FR, GB, IT, LI, NL, SE)

1. Surfactant mixtures which are composed of 20 to 80 % by weight of a $C_{12}$-$C_{20}$ α-olefinsulfonate and 20 to 80 % by weight of one or more compounds of the formulae

$$RCONH-(CH_2CH_2O)_n-SO_3X \quad (1)$$

in which R denotes $C_7$-$C_{17}$ alkyl, n denotes a number from 0 to 4 and X denotes an alkali metal ion, alkaline earth metal ion or alkanolammonium ion,

$$RCON(CH_3)-CH_2CH_2SO_3X \quad (2)$$

in which R and X have the same meaning as in formula (1),

$$R_1O(CH_2CH_2O)_nCOCH_2-CH(SO_3Y)-COOY \quad (3)$$

in which $R_1$ denotes $C_{12}$-$C_{14}$ alkyl, n denotes a number from 0 to 5 and Y denotes an alkali metal ion,

$$R_2CONH-(CH_2)_n-{}^{\oplus}N(R_3)_2-CH_2COO^{\ominus} \quad (4)$$

in which $R_2$ denotes $C_{10}$-$C_{14}$ alkyl, n denotes 2 or 3 and $R_3$ denotes $C_1$-$C_4$ alkyl,

$$R_4-\overset{\overset{R_3}{|}}{\underset{\underset{R_3}{|}}{N}}=O \qquad (5)$$

in which $R_4$ denotes $C_{10}$-$C_{18}$ alkyl and $R_3$ denotes $C_1$-$C_4$ alkyl, and

$$R_4CONH(CH_2)_n-\overset{\overset{R_3}{|}}{\underset{\underset{R_3}{|}}{N}}=O \qquad (6)$$

in which $R_4$ denotes $C_{10}$-$C_{18}$ alkyl, n denotes 2 or 3 and $R_3$ denotes $C_1$-$C_4$ alkyl.

2. Surfactant mixtures as claimed in Claim 1, which are composed of 30 to 70 % by weight of the α-olefinsulfonate and 30 to 70 % by weight of one or more compounds of the formulae (1) to (6).

**Claims** (for the Contracting State AT)

1. Process for the preparation of surfactant mixtures by mixing an α-olefinsulfonate with at least one further surfactant which process in characterized in that 20 to 80 % by weight of a $C_{12}$-$C_{20}$ α-olefinsulfonate are mixed with 20 to 80 % by weight of one or more compounds of the formulae

$$RCONH-(CH_2CH_2O)_n-SO_3X \qquad (1)$$

in which R denotes $C_7$-$C_{17}$ alkyl, n denotes a number from 0 to 4 and X denotes an alkali metal ion, alkaline earth metal ion or alkanolammonium ion,

$$RCO\overset{\overset{CH_3}{|}}{N}-CH_2CH_2SO_3X \qquad (2)$$

in which R and X have the same meaning as in formula (1),

$$R_1O(CH_2CH_2O)_nCOCH_2-\underset{\underset{SO_3Y}{|}}{CH}-COOY \qquad (3)$$

in which $R_1$ denotes $C_{12}$-$C_{14}$ alkyl, n denotes a number from 0 to 5 and Y denotes an alkali metal ion,

$$R_2CONH-(CH_2)_n-{}^{\oplus}\overset{\overset{R_3}{|}}{\underset{\underset{R_3}{|}}{N}}-CH_2COO^{\ominus} \qquad (4)$$

in which $R_2$ denotes $C_{10}$-$C_{14}$ alkyl, n denotes 2 or 3 and $R_3$ denotes $C_1$-$C_4$ alkyl,

$$R_4-\overset{\overset{R_3}{|}}{\underset{\underset{R_3}{|}}{N}}=O \qquad (5)$$

in which $R_4$ denotes $C_{10}$-$C_{18}$ alkyl and $R_3$ denotes $C_1$-$C_4$ alkyl, and

$$R_4CONH(CH_2)_n-\overset{\overset{R_3}{|}}{\underset{\underset{R_3}{|}}{N}}=O \qquad (6)$$

in which $R_4$ denotes $C_{10}$-$C_{18}$ alkyl, n denotes 2 or 3 and $R_3$ denotes $C_1$-$C_4$ alkyl.

2. A process as claimed in claim 1, which is characterized in that 30 to 70 % by weight of the α-olefinsulfonate are mixed with 30 to 70 % by weight of one or more compounds of the formulae (1) to (6).

**Revendications** (pour les Etats contractants : BE, CH, DE, FR, GB, IT, LI, NL, SE)

1. Mélanges tensio-actifs, caractérisés en ce qu'ils consistent en 20 à 80 % en poids d'un oléfine-alpha sulfonate en $C_{12}$ à $C_{20}$ et en 20 à 80 % en poids d'un ou plusieurs composés répondant aux formules

$$RCONH-(CH_2CH_2O)_n-SO_3X \qquad (1)$$

dans laquelle R représente un groupe alkyle en $C_7$ à $C_{17}$, n est un nombre valant 0 à 4 et X représente un ion de métal alcalin, de métal alcalino-terreux ou alcanolammonium,

$$RCO\overset{CH_3}{\overset{|}{N}}CH_2CH_2SO_3X \qquad (2)$$

dans laquelle R et X ont le même sens qu'à la formule (1)

$$R_1O(CH_2CH_2O)_nCOCH_2-\underset{SO_3Y}{\underset{|}{CH}}-COOY \qquad (3)$$

dans laquelle $R_1$ représente un groupe alkyle en $C_{12}$ à $C_{14}$, n est un nombre valant 0 à 5 et Y représente un ion de métal alcalin,

$$R_2CONH-(CH_2)_n-{}^{\oplus}\overset{R_3}{\underset{R_3}{N}}-CH_2COO^{\ominus} \qquad (4)$$

dans laquelle $R_2$ représente un groupe alkyle en $C_{10}$ à $C_{14}$, n vaut 2 ou 3 et $R_3$ représente un groupe alkyle en $C_1$ à $C_4$,

$$R_4-\overset{R_3}{\underset{R_3}{N}}=O \qquad (5)$$

dans laquelle $R_4$ représente un groupe alkyle en $C_{10}$ à $C_{18}$ et $R_3$ représente un groupe alkyle en $C_1$ à $C_4$, et

$$R_4CONH(CH_2)_n-\overset{R_3}{\underset{R_3}{N}}=O \qquad (6)$$

dans laquelle $R_4$ représente un groupe alkyle en $C_{10}$ à $C_{18}$, n vaut 2 ou 3 et $R_3$ représente un groupe alkyle en $C_1$ à $C_4$.

2. Mélanges tensio-actifs selon la revendication 1, caractérisés en ce qu'ils consistent en 30 à 70 % en poids de l'oléfine-alpha sulfonate et en 30 à 70 % en poids d'un ou plusieurs composés répondant aux formules (1) à (6).

**Revendications** (pour l'Etat contractant AT)

1. Procédé de préparation de mélanges tensio-actifs par mélangeage d'un oléfine-alpha sulfonate avec au moins un second tensio-actif, procédé caractérisé en ce qu'on mélange 20 à 80 % en poids d'un oléfine-alpha sulfonate en $C_{12}$ à $C_{20}$ avec 20 à 80 % en poids d'un ou plusieurs composés répondant aux formules

$$RCONH-(CH_2CH_2O)_n-SO_3X \qquad (1)$$

dans laquelle R représente un groupe alkyle en $C_7$ à $C_{17}$, n est un nombre valant 0 à 4 et X représente un ion de métal alcalin, de métal alcalin-terreux ou alcanolammonium,

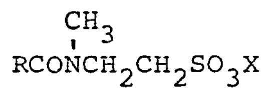

(2)

dans laquelle R et X ont le même sens que pour la formule (1),

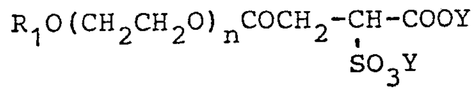

(3)

dans laquelle $R_1$ représente un groupe alkyle en $C_{12}$ à $C_{14}$, n est un nombre valant 0 à 5 et Y représente un ion de métal alcalin,

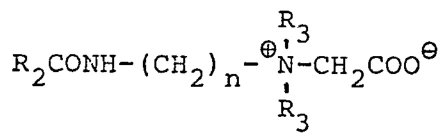

(4)

dans laquelle $R_2$ représente un groupe alkyle en $C_{10}$-$C_{14}$, n vaut 2 ou 3 et $R_3$ représente un groupe alkyle en $C_1$-$C_4$,

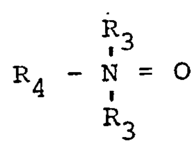

(5)

dans laquelle $R_4$ représente un groupe alkyle en $C_{10}$-$C_{18}$ et $R_3$ représente un groupe alkyle en $C_1$ à $C_4$ et

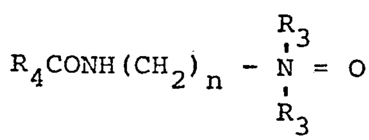

(6)

dans laquelle $R_4$ représente un groupe alkyle en $C_{10}$ à $C_{18}$, n vaut 2 ou 3, et $R_3$ représente un groupe alkyle en $C_1$ à $C_4$.

2. Procédé selon la revendication 1, caractérisé en ce qu'on mélange 30 à 70 % en poids de l'oléfine-alpha sulfonate avec 30 à 70 % en poids d'un ou plusieurs composés répondant aux formules 1 à 6.